# EUROPEAN PATENT SPECIFICATION

(11) **EP 3 378 975 B1**
(45) Date of publication and mention of the grant of the patent: **27.01.2021**
(21) Application number: 15908522.4
(22) Date of filing: 17.11.2015
(51) Int. Cl.: C40B 50/06, C40B 40/06, C12Q 1/6874, C12N 15/10

(54) **METHOD FOR BUILDING DNA LIBRARY FOR SEQUENCING**
VERFAHREN ZUM AUFBAU EINER DNA-BIBLIOTHEK ZUR SEQUENZIERUNG
PROCÉDÉ POUR CONSTRUIRE UNE BIBLIOTHÈQUE D'ADN POUR LE SÉQUENÇAGE

(43) Date of publication of application: 26.09.2018
(73) Proprietor: Zhejiang Annoroad Bio-Technology Co., Ltd., Choujiang Subdistrict Yiwu City, Zhejiang Province, 322000 (CN); Annoroad Gene Technology (Beijing) Co., Ltd, Beijing 100176 (CN)
(72) Inventor: CHEN, Chongjian, Beijing 100176 (CN); XIA, Yun, Beijing 100176 (CN); PENG, Qiongfang, Beijing 100176 (CN); WANG, Zhandong, Beijing 100176 (CN); XUE, Yuehan, Beijing 100176 (CN); XUAN, Zhaoling, Beijing 100176 (CN); LI, Dawei, Beijing 100176 (CN); LIANG, Junbin, Beijing 100176 (CN)
(74) Representative: Huenges, Martin
(86) International application number: PCT/CN2015/094786
(87) International publication number: WO 2017/084025

(56) References cited:
- WO-A1-2009/098037
- WO-A1-2014/071295
- WO-A2-2012/096579
- CN-A- 103 361 743
- CN-A- 104 099 666
- US-A1- 2012 329 678
- AUSTIN P. SO ET AL: "Minimizing loss of sequence information in SAGE ditags by modulating the temperature dependent 3'?->?5' exonuclease activity of DNA polymerases on 3'-terminal isoheptyl amino groups", BIOTECHNOLOGY AND BIOENGINEERING, vol. 94, no. 1, 5 May 2006 (2006-05-05), pages 54-65, XP055523510, US ISSN: 0006-3592, DOI: 10.1002/bit.20805
- J. KHATTRA ET AL: "Large-scale production of SAGE libraries from microdissected tissues, flow-sorted cells, and cell lines", GENOME RESEARCH, vol. 17, no. 1, 6 December 2006 (2006-12-06), pages 108-116, XP055523508, US ISSN: 1088-9051, DOI: 10.1101/gr.5488207 -& Jaswinder Khattra ET AL: "Large-scale production of SAGE libraries from microdissected tissues, flow-sorted cells, and cell lines Supplemental Research Data", Genome Res., 29 November 2006 (2006-11-29), pages 108-116, XP055523701, DOI: 10.1101/gr.5488207 Retrieved from the Internet: URL:https://genome.cshlp.org/content/17/1/ 108/suppl/DC1 [retrieved on 2018-11-14]
- STEVEN R. HEAD ET AL.: 'Library Construction for Next-Generation Sequencing: Overviews and Challenges' BIOTECHNIQUES vol. 56, no. 2, 01 February 2014, XP002740381

## Description

### FIELD OF THE INVENTION

The present invention pertains to the field of molecular biology, and in particular, relates to a method for constructing a DNA library for sequencing, a DNA library for sequencing constructed by the method, and the application of the DNA library for sequencing in sequencing.

### BACKGROUND OF THE INVENTION

In general, second-generation DNA sequencing is performed using a DNA library for sequencing containing double-stranded DNA for sequencing. In the double-stranded DNA for sequencing, a single DNA fragment to be sequenced is inserted between a 5' sequence and a 3' sequence, and the 5' sequence and the 3' sequence respectively contain a base sequence matched with a sequencing primer. Due to the limitations of the second-generation DNA sequencing technology, the length of the single DNA fragment to be sequenced is usually about 10 to 500 bp.

Moreover, as shown by So et al. (Biotechnol. Bioeng. Vol 94, Issue 1, 2006) the incorporation of short sequence tags (SSTs) into higher molecular weight (HMW) ligation products can lead to a loss of sequence information from SAGE analysis, potentially skewing sequencing results away from the true distribution in the original sample. By modifying fill-in conditions through the use of Vent® DNA polymerase (DNAP) at 12°C and by including terminal phosphorothioate linkages within the SAGE adaptors to specifically inhibit exonucleolytic removal of the 3'-terminal amine it was possible to maximize the yield of ditags and bypass the need for gel purification via PAGE following PCR. In the second-generation DNA sequencing using the aforementioned DNA library for sequencing, it is necessary to read data separately for each double-stranded DNA for sequencing which contains a single DNA fragment to be sequenced, that is, the data of only one DNA fragment to be sequenced can be obtained in one sequencing process, although the existing sequencers can already read data from both ends of the double-stranded DNA simultaneously. When this conventional DNA library for sequencing is used for sequencing, the dosage of sequencing reagents is large and the time for sequencing is long, resulting in a high cost and a low efficiency of sequencing.

### SUMMARY OF THE INVENTION

In view of the above deficiencies existing in the prior art, an object of the present invention is to provide a method for constructing a DNA library for sequencing and a sequencing method using a DNA library for sequencing constructed using the method for constructing a DNA library for sequencing, wherein, in the sequencing using a DNA library for sequencing constructed using the method for constructing a DNA library for sequencing, the dosage of sequencing reagents can be reduced and the time for sequencing can be shortened, thereby reducing the sequencing cost and improving the sequencing efficiency.

The inventors of the present invention conducted intensive studies in order to solve the above technical problems, and as a result, they have found that by changing the step of A-tailing of double 3' ends into a step of A-tailing of a single 3' end and adding a step of blunt-end ligation in the conventional method for constructing a DNA library for sequencing, it is possible to insert two or more DNA fragments to be sequenced between the 5' sequence and the 3' sequence of the double-stranded DNA for sequencing, which makes it possible to simultaneously read the data of two DNA fragments to be sequenced from both ends of the double-stranded DNA in the sequencing step, thereby reducing the dosage of sequencing reagents and shortening the time for sequencing.

That is, the present invention based on the findings aforementioned includes:
1. A method for constructing a DNA library for sequencing, comprising:
   step B-1: A-tailing of a single 3' end to at least a portion of a blunt-end DNA fragment to obtain a DNA fragment having A tailing to the single 3' end; and
   step B-2: blunt-end ligating the DNA fragments with an A-tail at the single 3' end obtained in step B-1.
2. The method for constructing a DNA library for sequencing according to item 1, further comprising:
   step A performed before the step B: end-repairing DNA fragments to be sequenced to obtain blunt-end DNA fragments;
   step C performed after the step B-2 or simultaneously with the step B-2: adding adapters to the blunt-end ligated products of the step B-2 to obtain adapter-added DNA fragments; and
   step D performed after the step C: subjecting the adapter-added DNA fragments to PCR amplification to obtain amplification products.
3. The method for constructing a DNA library for sequencing according to item 1 or 2, further comprising:
   step E performed after the step D: fragment selection of the amplification products.
4. The method for constructing a DNA library for sequencing according to any one of items 1 to 3, further comprising:
   step F performed after the step E: purifying the amplification products after the fragment selection.
5. The method for constructing a DNA library for sequencing according to any one of items 1 to 4, further comprising:
   step C1 performed after the step C: fragment selection of the adapter-added DNA fragments; and in the step D, subjecting the adapter-added DNA fragments after the fragment selection to PCR amplification to obtain amplification products.
6. The method for constructing a DNA library for sequencing according to any one of items 3 to 5, wherein the amplification products containing two or more DNA fragments to be sequenced is selected.
7. A DNA library for sequencing obtained by the method of any one of items 1 to 6, wherein double-stranded DNAs containing two or more DNA fragments to be sequenced make up at least 30 mol% of the total double-stranded DNAs.
8. A sequencing method, wherein sequencing is conducted taking the DNA library for sequencing according to item 7 as an object.
9. The sequencing method according to item 8, wherein the sequencing is paired-end sequencing.

### EFFECT OF THE INVENTION

In the double-stranded DNAs for sequencing contained in the DNA library for sequencing constructed using the method for constructing a DNA library for sequencing, two or more DNA fragments to be sequenced are allowed to be inserted between the 5' sequence and the 3' sequence, which makes it possible to read data from both ends of the double-stranded DNAs for sequencing simultaneously in one sequencing process in case of using a paired-end sequencing mode, and thus the dosage of sequencing reagents can be reduced and the time for sequencing can be shortened, thereby reducing the sequencing cost and improving the sequencing efficiency. In addition, the technology of A-tailing of a single 3' end saves the reagents used to construct libraries and reduces the cost of constructing DNA libraries and sequencing.

### DESCRIPTION OF DRAWINGS

Fig. 1 is a comparison diagram between the library construction process of the present invention and a conventional library construction process;
Fig. 2 is a schematic diagram of results of DNA library fragments sizes testing using Agilent 2100 Bioanalyzer of Example 2;
Fig. 3 is a diagram of a double-end sequencing base distribution of Example 3;
Fig. 4 is a diagram of a single-end sequencing base distribution of Example 3;
Fig. 5 is a diagram of a double-end sequencing mass distribution of Example 3;
Fig. 6 is a diagram of a single-end sequencing mass distribution of Example 3.

### DETAILED DESCRIPTION OF THE INVENTION

First, in one aspect, the present invention provides a method for constructing a DNA library for sequencing (the method for constructing a DNA library for sequencing of the present invention), comprising:
step B-1: A-tailing of a single 3' end for a blunt-end DNA fragment to obtain a DNA fragment having A tailing to the single 3' end; and
step B-2: blunt-end ligating the DNA fragment having A tailing to the single 3' end obtained in step B-1.

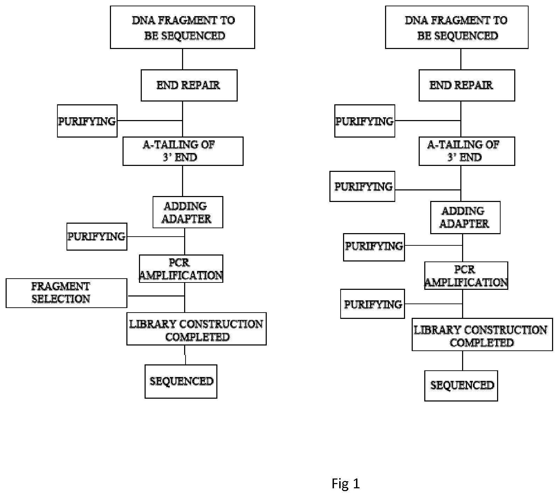

As shown in the right diagram of Fig. 1, in contrast to the conventional method for constructing a DNA library for sequencing where blunt-end DNA fragments obtained by end repair is added with A bases to double 3' ends, in the method for constructing a DNA library for sequencing of the present invention, blunt-end DNA fragments obtained by end repair is subjected to A-tailing of a single 3' end to obtain DNA fragments having A tailing to a single 3' end, and moreover, the obtained DNA fragments with A tailing to a single 3' end are blunt-end ligated in the method for constructing a DNA library for sequencing of the present invention. Through the above steps, the DNA fragments containing two or more DNA fragments to be sequenced obtained by blunt-end ligation can be inserted as a whole between the 5' sequence and the 3' sequence of the double-stranded DNA for sequencing in a subsequent step.

The A-tailing of a single 3' end of the blunt-end DNA fragment described above can be achieved by any method known to the skilled person in the art, for example, by using DNA polymerases having the above function (e.g., Klenow fragment lacking 3' end to 5' end exonuclease activity). The skilled person in the art can easily achieve A-tailing of a single 3' end by reducing the dosage of enzyme as used, lowering the reaction temperature, and / or shortening the reaction time based on the conventional enzyme reaction of A-tailing of double 3' ends.

The blunt-end ligation of the DNA fragments having A tailing to a single 3' end described above can be achieved by any method known to the skilled person in the art, for example, by using DNA ligases with a blunt end ligation function (e.g., T4 DNA ligase, T3 DNA ligase).

Preferably, the method for constructing a DNA library for sequencing of the present invention further comprises:
step A performed before the step B: end-repairing DNA fragments to be sequenced to obtain blunt-end DNA fragments;
step C performed after the step B-2 or simultaneously with the step B-2: adding adapters to the blunt-end ligated products of the step B-2 to obtain adapter-added DNA fragments; and
step D performed after the step C: subjecting the adapter-added DNA fragments to PCR amplification to obtain amplification products.

The above steps A, C and D can be performed by conventional methods in the technical field. It should be noted that since step B-2 and step C can be performed by using some DNA ligases (e.g., T4 DNA ligase, T3 DNA ligase), step B-2 and step C can be performed simultaneously by using the aforementioned DNA ligases (e.g., T4 DNA ligase, T3 DNA ligase).

The "DNA fragments to be sequenced" in step A is not particularly limited and is preferably DNA fragments about 10 to 1000 bp, more preferably about 20 to 800 bp, more preferably about 30 to 750 bp, more preferably about 40 to 700 bp, more preferably about 50 to 650 bp, more preferably about 100 to 600 bp, more preferably about 150 to 550 bp, from a sequencer acceptance perspective.

Preferably, the method for constructing a DNA library for sequencing of the present invention further comprises step E performed after the step D: fragment selection of the amplification products.

Preferably, step C1 performed after the step C: fragment selection of the adapter-added DNA fragments; and in the step D, the adapter-added DNA fragments after the fragment selection is subjected to PCR amplification to obtain amplification products.

In the step C1 and the step E, it is preferable to select the amplification products in which two or more DNA fragments to be sequenced are inserted (that is, to remove as much as possible amplification products in which only one DNA fragment to be sequenced is inserted, or amplification products in which no DNA fragment to be sequenced is inserted).

The fragment selection described above may be performed by a conventional method in the technical field, for example, by using magnetic beads. In the case of using magnetic beads for fragment selection, the dosage of the magnetic beads can be adjusted according to the size of the amplification products to be selected, for example, so as to obtain the specific amplification products.

Preferably, a step of purifying DNA fragments may be added between each step of the method for constructing a DNA library for sequencing of the present invention, for example, between step A and step B-1, between step B-1 and step C, between step C and step C1, between step C (or C1) and step D, between step D and step E, and / or after step E.

This purification step can be carried out by using a conventional method in the technical field, for example, by using purified magnetic beads.

The method for constructing a DNA library for sequencing of the present invention is significantly different from the conventional method in that it seeks to integrate two or more DNA fragments to be sequenced in a double-stranded DNA for sequencing, while the conventional method avoids such situation.

Therefore, in one aspect, the present invention provides a DNA library for sequencing (the DNA library for sequencing of the present invention), wherein the ratio of the double-stranded DNAs containing two or more DNA fragments to be sequenced to the total double-stranded DNAs is notably higher than that in a conventional DNA library for sequencing. This ratio may be, for example, at least 10 mol%, preferably at least 20 mol%, more preferably at least 30 mol%, more preferably at least 40 mol%, more preferably at least 50 mol%, more preferably at least 60 mol%, more preferably at least 70 mol%, more preferably at least 80 mol%, more preferably at least 90 mol%.

The DNA library for sequencing of the present invention is constructed by using the method for constructing a DNA library for sequencing of the present invention, for example.

In addition, in one aspect, the present invention provides a sequencing method (the sequencing method of the present invention), wherein sequencing is conducted taking the DNA library for sequencing of the present invention as an object.

In addition to taking the DNA library for sequencing of the present invention as an object, the sequencing method of the present invention may be conducted using a conventional method in the technical field. Preferably, the sequencing method of the present invention may employ a paired-end sequencing, for example, a paired-end sequencing using Illumina platform (e.g., HiSeq 2500 or NextSeq 500).

### EXAMPLES

The present invention will be described more specifically below with examples. It should be understood that the examples described herein are intended to explain the present invention, rather than to limit the present invention.

### Example 1 Constructing a DNA library for sequencing using the method of the present invention

### (1) Preparation of DNA fragments to be sequenced

The DNA fragments to be sequenced used in the present example are a mixture of some DNA fragments about 300 bp and some DNA fragments about 180 bp. Some of the double-stranded DNA fragments are blunt-ended, and the others contain 3' or 5' sticky end.

### (2) End repairing to obtain blunt-end DNA fragments

A. The reaction system is as follows:

| Reagent | Volume |
|---|---|
| DNA fragments obtained in step (1) | 42 µL |
| Mixture of dNTPs (10mM) | 1 µL |
| T4 DNA polymerase | 1 µL |
| T4 PNK (T4 polynucleotide kinase) | 1 µL |
| PNK buffer | 5 µL |
| In total | 50 µL |

B. Incubating in a PCR instrument for 30 minutes at a temperature of 20 °C;
C. Purifying the reaction product by magnetic beads, eluting with 19.5 µL of elution buffer EB (Elution Buffer) to obtain blunt-end DNA fragments.

### (3) A-tailing of a single 3' end to blunt-end DNA fragments

The reaction system is as follows:

| Reagent | Volume |
|---|---|
| DNA fragments obtained in step (2) | 19.5 µL |
| Klenow buffer (10 ×) | 2.5 µL |
| dATP (1 mM) | 2.5 µL |
| Klenow fragments (lacking 3' to 5' exonuclease activity) | 0.05 µL |
| ddH₂O | 0.45 µL |
| In total | 25 µL |

They were incubated for 30 minutes in a PCR instrument at a temperature of 37 °C.

### (4) Specific adapter ligation and purification;

In order to perform specific amplification in PCR, it is necessary to add a specific adapter to both ends of the fragments having A tailing to the single 3' end obtained in (3), and the adapter sequences required for the ligation reaction need to be mixed in equal proportion when in use. The adapter sequences are as follows:
Adapter sequence 1: 5' P-GATCGGAAGAGCACACGTCT-3'
Adapter sequence 2: 5' P-ACACTCTTTCCCTACACGACGCTCTTCCGATCT-3'
A. The reaction system is as follows:

| Reagent | Volume |
|---|---|
| DNA fragments obtained in step (3) | 25 µL |
| DNA ligase buffer (2 ×) | 25 µL |
| T4 DNA ligase (1 U/µL) | 1 µL |
| Adapter (0.04 pmol/µL) | 5 µL |
| In total | 52 µL |

B. Incubating in a PCR instrument for 15 minutes at a temperature of 20 °C;
C. Purification - purifying the reaction products by magnetic beads, eluting with 19 µL of elution buffer EB (Elution Buffer) to obtain DNA fragments with adapters at both ends.

It should be noted that the fragments having A tailing to a single 3' end obtained in (3) were subjected to blunt end ligation in this step.

### (5) PCR amplification and purification of DNA fragments with both ends ligated to adapters

The DNA fragments having adapters added to both ends were subjected to PCR amplification to add base sequences matched with a sequencing primer to both ends of the DNA fragments in the process of PCR on the one hand, and to ensure that there is enough total amount of DNA fragments for the subsequent sequencing on the other hand.

Primer sequences used in PCR amplification:
PCR Primer 1: 
5'-
AATGATACGGCGACCACCGAGATCTACACTCTTTCCCTACACGACGCTCTTCCGATCT-3'
PCR Primer 2: 
5'-
CAAGCAGAAGACGGCATACGAGATCGTGATGTGACTGGAGTTCAGACGTGTGCTCTTCC
GATCT-3'
A. The reaction system is as follows:

| Reagent | Volume |
|---|---|
| DNA fragments having adapters added to both ends | 17 µL |
| 2 × DNA polymerase amplification mixture | 25 µL |
| PCR Primer 1 (10 pmol/µL) | 4 µL |
| PCR Primer 2 (10 pmol/µL) | 4 µL |
| In total | 50 µL |

B. After adding samples, placing in PCR and holding at 94 °C for 2 minutes; (94 °C for 15 seconds; 62 °C for 30 seconds; 72 °C for 30 seconds) 17 cycles; then 72 °C for 10 minutes; and finally storing the sample at 4 °C.
C. Fragment selection and purification

The product obtained from the reaction was subjected to fragment selection and purification by magnetic beads (steps are described below) and was eluted with a certain amount of elution buffer EB (Elution Buffer), thereby completing the preparation of a DNA library for sequencing. The DNA library for sequencing was stored in a suitable environment for further use.
a. AmPure magnetic beads were equilibrated for 30 minutes at room temperature.
b. According to the number of samples to be purified, an equal number of 1.5 mL EP tubes were labeled as EP tubes.
c. The well-equilibrated magnetic beads of a were vortex blended, and 32.5 µL of magnetic beads were dispensed in the 1.5 mL EP tubes of b.
d. The PCR products and the magnetic beads of c were mixed and blended by mixing and rested at room temperature for 5 minutes. Then they were moved to the magnetic frame and stood for 3 minutes, and then the supernatant was discarded.
e. The 1.5 mL EP tubes of d were moved to the magnetic frame to stand for 3 minutes, and the supernatant was discarded.
f. 300 µL of 70% ethanol was added to the 1.5 mL EP tubes and the tubes were repeatedly mixed uniformity, and then the supernatant was discarded. This step was repeated. Then ethanol was absorbed clean and the tubes were allowed to dry at room temperature for 5 minutes.
g. The 1.5 mL EP tubes were moved from the magnetic frame to the EP tube holder and each EP tube was added with 52 µL of elution buffer and was mixed uniformity. The tubes were placed at room temperature for 5 minutes and then were moved to the magnetic frame standing for 1 minute.
h. The supernatant is the DNA library obtained after the fragment selection and purification. The supernatant was transferred to a new EP tube for direct sequencing or to store in a freezer of - 20 °C.

### Example 2 Examining the quality of the DNA library for sequencing

The fragment sizes of the DNA library constructed in Example 1 were tested using Agilent 2100 Bioanalyzer.

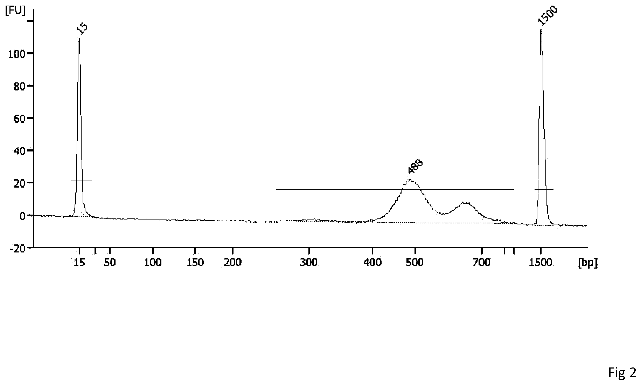

It is possible to know whether the constructed library fragments meet the above quality standards of the library by using Agilent 2100 Bioanalyzer to test the library. The test results are shown in Fig. 2. It can be seen from Fig. 2 that
(1) there are mainly two different sizes of fragments, 480 bp ± 10% and 660 bp ± 10%;
(2) the proportion of fragments of 480 bp ± 10% is about 50% to 70%, and the proportion of fragments of 660 bp ± 10% is about 30% to 50%.

This shows that the DNA library obtained in Example 1 is qualified.

### Example 3 Sequencing the DNA library for sequencing using Illumina NextSeq 500

The DNA library for sequencing constructed in Example 1 was conducted by single-end sequencing and paired-end sequencing respectively, and the reagent used for sequencing was NextSeq™ 500 High Output v2 Kit. The sequencing results are shown in Fig.s 3 to 6.

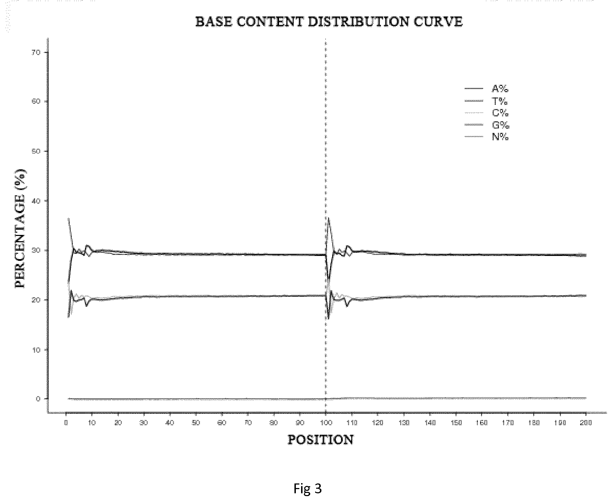

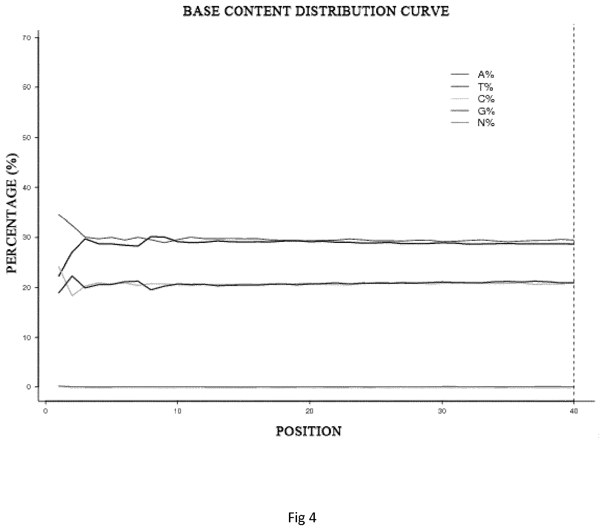

As can be seen from the base distribution diagrams of Fig. 3 and Fig. 4, the result of single-end sequencing of the above mentioned DNA library for sequencing and the result of paired-end sequencing of the above mentioned DNA library for sequencing are basically the same.

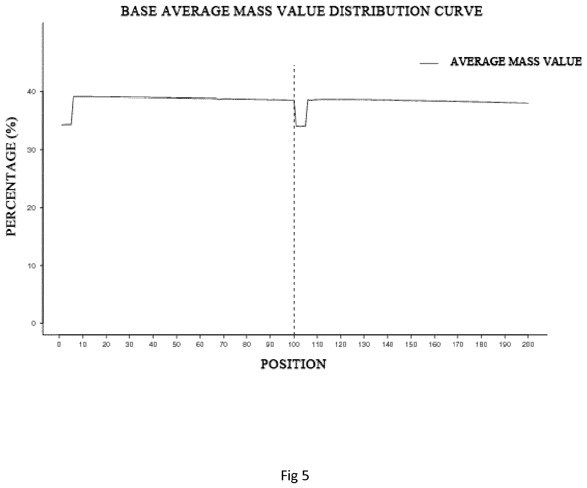

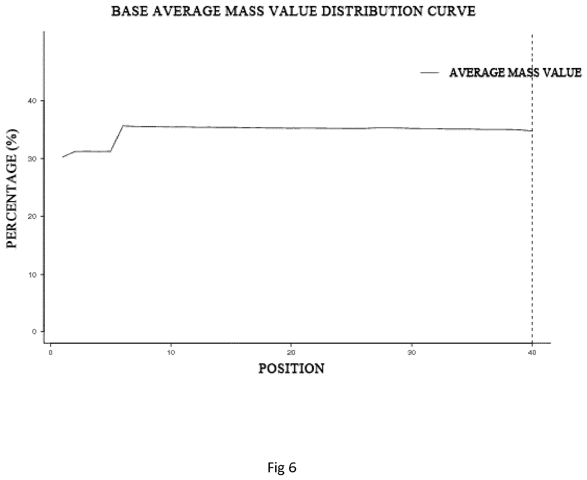

As can be seen from the mass distribution diagrams of Fig. 5 and Fig. 6, the mass curves are smooth and do not have falling points.

It should also be noted that any one of the technical features or combinations of the technical features described as constituents of a technical solution in the present specification may also be applied to other technical solutions on the premise that they can be practiced and do not contradict the gist of the present invention; moreover, the technical features described as constituents of different technical solutions may also be combined in any manner to form other technical solutions on the premise that they can be practiced and do not contradict the gist of the present invention. The present invention also includes technical solutions obtained by combinations in the aforementioned cases, and these technical solutions are regarded as being described in the present specification.

The above description shows and describes preferred examples of the present invention. As aforementioned, it should be understood that the present invention is not limited to the forms disclosed herein, and should not be construed as an exclusion of other examples, but may be applied to various other combinations, modifications and environments, and may be altered within the scope of the inventive concepts described herein by the above teachings or techniques or knowledge in related arts.

### INDUSTRIAL APPLICABILITY

The sequencing cost can be reduced and the sequencing efficiency can be improved in the sequencing using a DNA library for sequencing constructed by the method of constructing a DNA library for sequencing of the present invention.

## Claims

1. A method for constructing a DNA library for sequencing, comprising:
step B-1: A-tailing of a single 3' end to at least a portion of blunt-end DNA fragments to obtain DNA fragments having A tailing to the single 3' end; and
step B-2: blunt-end ligating the DNA fragments having A tailing to the single 3' end obtained in step B-1.

2. The method for constructing a DNA library for sequencing according to claim 1, further comprising:
step A performed before the step B: end-repairing the DNA fragments to be sequenced to obtain blunt-end DNA fragments;
step C performed after the step B-2 or simultaneously with the step B-2: adding adapters to the blunt-end ligated products of the step B-2 to obtain adapter-added DNA fragments; and
step D performed after the step C: subjecting the adapter-added DNA fragments to PCR amplification to obtain amplification products.

3. The method for constructing a DNA library for sequencing according to claim 2, further comprising:
step E performed after the step D: fragment selection of the amplification products.

4. The method for constructing a DNA library for sequencing according to claim 3, further comprising:
step F performed after the step E: purifying the amplification products after the fragment selection.

5. The method for constructing a DNA library for sequencing according to claim 2, further comprising:
step C1 performed after the step C: fragment selection of said adapter-added DNA fragments;
and in the step D, subjecting the adapter-added DNA fragments after the fragment selection to PCR amplification to obtain amplification products.

6. The method for constructing a DNA library for sequencing according to any one of claims 3 to 5, wherein amplification products containing two or more DNA fragments to be sequenced is selected.

7. A DNA library for sequencing obtained by the method of any one of claims 1 to 6, wherein double-stranded DNAs containing two or more DNA fragments to be sequenced make up at least 30 mol% of the total double-stranded DNAs.

8. A sequencing method, wherein sequencing is conducted taking the DNA library for sequencing according to claim 7 as an object.

9. The sequencing method according to claim 8, wherein the sequencing is paired-end sequencing.

## Patentansprüche

1. Verfahren zum Aufbau einer DNA-Bibliothek zur Sequenzierung, umfassend:
Schritt B-1: A-Tailing eines einzelnen 3'-Endes an mindestens einen Teil von DNA-Fragmenten mit stumpfem Ende, um DNA-Fragmente mit A-Tailing an dem einzelnen 3'-Ende zu erhalten; andere Schritt B-2: Blunt-End-Ligieren der DNA-Fragmente mit A-Tailing an das in Schritt B-1 erhaltene einzelne 3'-Ende.

2. Verfahren zum Aufbau einer DNA-Bibliothek zur Sequenzierung nach Anspruch 1, ferner umfassend:
Schritt A, der vor dem Schritt B durchgeführt wird: Endreparatur der zu sequenzierenden DNA-Fragmente, um DNA-Fragmente mit stumpfem Ende zu erhalten;
Schritt C, der nach dem Schritt B-2 oder gleichzeitig mit dem Schritt B-2 durchgeführt wird: Hinzufügen von Adaptern zu den blunt-end-ligierten Produkten des Schritts B-2, um adapteraddierte DNA-Fragmente zu erhalten; und
Schritt D, der nach Schritt C durchgeführt wird: Unterziehen der adapteraddierten DNA-Fragmente einer PCR-Amplifikation, um Amplifikationsprodukte zu erhalten.

3. Verfahren zum Aufbau einer DNA-Bibliothek zur Sequenzierung nach Anspruch 2, ferner umfassend:
Schritt E, der nach dem Schritt D durchgeführt wird: Fragmentauswahl der Amplifikationsprodukte.

4. Verfahren zum Aufbau einer DNA-Bibliothek zur Sequenzierung nach Anspruch 3, ferner umfassend:
Schritt F, der nach dem Schritt E durchgeführt wird: Reinigen der Amplifikationsprodukte nach der Fragmentauswahl.

5. Verfahren zum Aufbau einer DNA-Bibliothek zur Sequenzierung nach Anspruch 2, ferner umfassend:
Schritt C1, der nach dem Schritt C durchgeführt wird: Fragmentauswahl der adapteradditierten DNA-Fragmente; und in Schritt D Unterziehen der adapteradditierten DNA-Fragmente einer PCR-Amplifikation nach der Fragmentauswahl, um Amplifikationsprodukte zu erhalten.

6. Verfahren zum Aufbau einer DNA-Bibliothek zur Sequenzierung nach einem der Ansprüche 3 bis 5, wobei Amplifikationsprodukte, die zwei oder mehr zu sequenzierende DNA-Fragmente enthalten, ausgewählt werden.

7. DNA-Bibliothek zur Sequenzierung, erhalten durch das Verfahren nach einem der Ansprüche 1 bis 6, wobei doppelsträngige DNAs, die zwei oder mehr zu sequenzierende DNA-Fragmente enthalten, mindestens 30 Mol-% der gesamten doppelsträngigen DNAs ausmachen.

8. Sequenzierungsverfahren, wobei eine Sequenzierung unter Verwendung der DNA-Bibliothek zur Sequenzierung gemäß Anspruch 7 als ein Objekt durchgeführt wird.

9. Sequenzierungsverfahren nach Anspruch 8, wobei die Sequenzierung eine Paired-End-Sequenzierung ist.

## Revendications

1. Procédé de construction d'une banque d'ADN pour séquençage, comprenant :
étape B-1 : adénylation d'une extrémité 3' unique à au moins une partie de fragments d'ADN à extrémités franches pour obtenir des fragments d'ADN à bout A à l'extrémité 3' unique ; et
étape B-2 : ligature d'extrémités franches des fragments d'ADN à bout A à l'extrémité 3' unique obtenus à l'étape B-1.

2. Le procédé de construction d'une banque d'ADN pour séquençage selon la revendication 1, comprenant en outre :
étape A effectuée avant l'étape B : réparation d'extrémités des fragments d'ADN à séquencer pour obtenir des fragments d'ADN à extrémités franches ;
étape C effectuée après l'étape B-2 ou simultanément à l'étape B-2 : ajout d'adaptateurs aux produits ligaturés à extrémités franches de l'étape B-2 pour obtenir des fragments d'ADN à adaptateurs ajoutés ; et
étape D effectuée après l'étape C : soumission des fragments d'ADN à adaptateurs ajoutés à une amplification PCR pour obtenir des produits d'amplification.

3. Le procédé de construction d'une banque d'ADN pour séquençage selon la revendication 2, comprenant en outre :
étape E effectuée après l'étape D : sélection de fragments des produits d'amplification.

4. Le procédé de construction d'une banque d'ADN pour séquençage selon la revendication 3, comprenant en outre :
étape F effectuée après l'étape E : purification des produits d'amplification après la sélection de fragments.

5. Le procédé de construction d'une banque d'ADN pour séquençage selon la revendication 2, comprenant en outre :
étape C1 effectuée après l'étape C : sélection de fragments desdits fragments d'ADN à adaptateurs ajoutés ;
et à l'étape D, soumission des fragments d'ADN à adaptateurs ajoutés après la sélection de fragments à une amplification PCR pour obtenir des produits d'amplification.

6. Le procédé de construction d'une banque d'ADN pour séquençage selon l'une quelconque des revendications 3 à 5, sachant que des produits d'amplification contenant deux ou plus de deux fragments d'ADN à séquencer sont sélectionnés.

7. Banque d'ADN pour séquençage obtenue par le procédé de l'une quelconque des revendications 1 à 6, sachant que des ADN à double brin contenant deux ou plus de deux fragments d'ADN à séquencer représentent au moins 30 % mol. du total des ADN à double brin.

8. Procédé de séquençage, sachant que le séquençage est effectué en prenant pour objet la banque d'ADN pour séquençage selon la revendication 7.

9. Le procédé de séquençage selon la revendication 8, sachant que le séquençage est un séquençage par paires d'extrémités.
